(19) Europäisches Patentamt
European Patent Office
Office européen des brevets

(11) Publication number: 0 181 068 A2

# (12) EUROPEAN PATENT APPLICATION

(21) Application number: 85306468.1

(22) Date of filing: 11.09.85

(51) Int. Cl.[4]: C 02 F 1/50

(30) Priority: 09.11.84 GB 8428374

(43) Date of publication of application: 14.05.86 Bulletin 86/20

(84) Designated Contracting States: DE FR GB IT NL SE

(71) Applicant: DEARBORN CHEMICAL COMPANY
300 Genesee Street
Lake Zurich IL 60047(US)

(72) Inventor: Clifford, Richard Purdie
19 Berry Drive
Great Sutton South Wirral(GB)

(72) Inventor: Clarkson, Douglas
64 Long Lane
Authton Lancashire(GB)

(72) Inventor: Longworth, Stuart Wilson
45 Mapplewell Crescent
Warrington Cheshire(GB)

(74) Representative: Ellis-Jones, Patrick George Armine et al,
J.A. KEMP & CO. 14 South Square Gray's Inn
London WC1R 5EU(GB)

## (54) Halogen release biocide.

(57) A method of treating an aqueous system to reduce bacteria therein is disclosed which comprises applying a method for the treatment of an aqueous system which comprises applying thereto an oxazolidinone of the formula

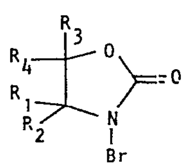

wherein $R_1$, $R_2$, $R_3$ and $R_4$, which may be the same or different, each represents a hyarogen atom or an alkyl radical of one to six atoms. There is also disclosed a process for making these oxazolidinones which comprises reacting an alkaline solution of the corresponding unbrominated compound with a solution of bromine in a water immiscible solvent.

EP 0 181 068 A2

DESCRIPTION

HALOGEN RELEASE BIOCIDE

This invention relates to the treatment of aqueous systems.

In industrial cooling water systems, for instance in industrial cooling towers, the water used is not, of course, sterile with the result that bacteria accumulate in the system and this quite commonly gives rise to a slimy deposit on the surfaces of the system which come into direct contact with the cooling water. A similar situation applies in paper making; slime can deposit on any of the surfaces with which the water comes into contact including the paper pulping bath, on the paper web and in the recirculating back water pipework. A large variety of different treating agents have been used for the purposes of killing these bacteria and/or inhibiting slime formation. These chemicals include quaternary ammonium compounds, amines, isothiazolones, chlorine, phenols, chlorine release agents, methylene-bis (thiocyanates), certain aldehydes and tin compounds.

The halogens, chlorine and bromine, are effective agents but suffer from the disadvantage of being toxic.

Accordingly, various compounds have been devised which will release chlorine in the water. It has now been

found that certain bromine compounds act as effective biocides in water. These compounds are brominated oxazolidinones.

According to the present invention there is provided a method for the treatment of an aqueous system which comprises applying thereto a compound of the formula

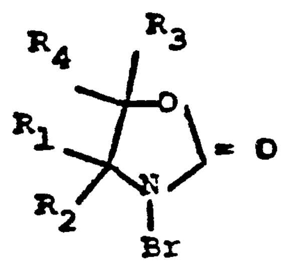

wherein $R_1$, $R_2$, $R_3$ and $R_4$, which may be the same or different, each represents a hydrogen atom or an alkyl radical of one to six carbon atoms, especially a methyl radical.

Preferably, the compounds used are ones in which $R_1$ and $R_2$ both represent a said alkyl radical. A preferred compound is 3-bromo-4,4-dimethyl-oxazolidin-2-one.

The oxazolidinones used in the present invention can be prepared, according to another aspect of the present invention, by reacting an alkaline solution of the corresponding unbrominated compound with a solution of bromine in a water-immiscible solvent. It has been found that the use of this heterogeneous reaction system gives significantly better yields than those previously recorded where no organic water immiscible solvent is employed.

The preferred solvent for the bromine is carbon tetrachloride although other solvents which may be used

include carbon tetrabromide, bromoform, benzene, n-hexane, 1,1,2,2,-tetrachloroethane, dichloromethane, chloro, bromo-methane, 1,2-dibromoethane, chloroform, carbon disulphide, toluene, 1,1,1-trichloroethane, diethyl ether, dibromomethane and nitrobenzene.

Typically, a solution of the unbrominated oxazolidinone in sodium hydroxide is prepared and cooled to, say,+5$^{o}$ to -5$^{o}$C, especially about 0$^{o}$C. A solution of bromine in the organic solvent is then added with cooling to keep the temperature from rising, typically with the aid of an ice/water bath with agitation, typically using a mechanical stirrer to provide fairly vigorous stirring, whereupon the desired material precipitates out. Normally precipitation occurs almost immediately but, in general, agitation is continued for up to, say, 30 minutes after the addition. Approximately stoichiometric quantities of about one mole of each reactant per one mole of the other should generally be used; about two moles of sodium hydroxide should be used per one mole of each reactant. Preferably the solutions used should be as concentrated as possible, typically saturated. Thus in general the oxazolidinone solution will contain about 0.5 to 4.5 moles of the oxazolidinone per litre while the bromine solution will contain about 1 to 10 moles of bromine per litre and the sodium hydroxide solution about 1 to 9 moles of sodium hydroxide per litre.

This precipitate can then be purified, if desired, by washing with a bromine solvent such as those mentioned above, preferably the solvent used for the bromine, to remove unreacted starting material, again with cooling of the solvent to, say, +5° to -5°C, especially about 0°C, to avoid dissolving the desired product. Alternatively the solutions of bromine and oxazolidinone can be mixed and the mixture cooled, or a solution of the oxazolidinone added to a solution of bromine, although this generally gives less good results and, in particular, a lower yield.

The brominated oxazolidinones used in the present invention dissolve slowly in water as an effective biocide. It is believed that the biocidal activity is caused by the hypobromous acid formed by the reaction

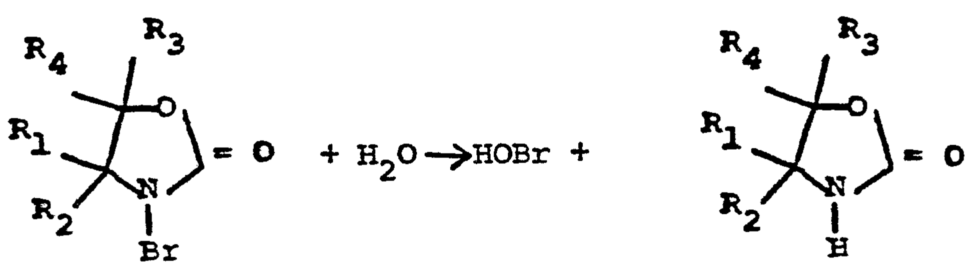

The brominated oxazolidinones generally take the form of a powder and are suitably added to the water either alone or with a solid filler, such as sodium sulphate, but typically in the form of pellets or tablets. Whether or not filler is included and the amount of filler included depends on the desired rate of release of the brominated compound. Naturally, the rate of release desired depends on the nature and amount of the bacteria present but, clearly, an amount effective to control the bacteria present should be used. Desirably a reserve of free hypobromous acid should be maintained in the system, typically in an amount of 0.2 to 100 ppm, preferably 1 to 10 ppm. Thus the oxazolidinone should be added in an amount which is typically from 0.2 to 500 ppm, preferably 1 to 50 ppm.

In paper making, the brominated compound may be

added directly to the paper pulping bath, the recirculating back water or to a holding tank containing, generally moist, pulp along with one or more chemical additives used in paper making. Such additives include starch, for example potato or corn starch, titanium dioxide, a de-foamer such as a fatty acid alcohol, a size, for example a rosin size based on abietic acid, a neutral size based on alkyl ketene dimer or a succinic acid anhydride based size, a wet strength resin such as, if neutral, an epichlorohydrin polyamide or, if acid, a melamine- or urea-formaldehyde resin, various polymers used as dispersants or retention aids such as polyacrylates, polymethacrylates, polyamides and polyacrylamides, clay, chalk, fillers such as carboxymethylcellulose, polyvinyl alcohol and optical brightening agents. In cooling water systems the material is preferably in stick or tablet form and placed in the system so that the water to be treated flows over it thus releasing the active ingredient or hypobromous acid. One way of achieving this slow dissolution is to suspend the sticks or tablets in a mesh bag in the recirculating water. Another way is to place the sticks or tablets in a separate unit through which flows a proportion of the cooling water feed or system water, thus dissolving the material and introducing free hypobromous acid into the system.

The following examples illustrate the preparation and utility of the brominated compounds in the present invention.

Example 1

Synthesis 3-bromo-4, 4-dimethyl-oxazolidin-2-one

A solution of 4,4-dimethyl-oxazolidin-2-one (11.9g),

prepared according to the method of Homeyer (U.S. Patent 2,399,118), and sodium hydroxide (6.0 g) in water (25 $cm^3$) was cooled to $0^oC$. To this was added, with vigorous stirring, a solution of bromine (16.0 g) in carbon tetrachloride (10 $cm^3$), keeping the temperature at $0^oC$. After stirring at this temperature for 30 minutes the precipitated product was filtered off, washed with ice-cold carbon tetrachloride and dried. A yield of 18.6g (92%) of 3-bromo-4,4-dimethyl-

oxazolidin-2-one (mp 118 - $119^oC$) as a pale yellow solid, was obtained.

Example 2

Solubility of 3-bromo-4,4-dimethyl oxazolidin-2-one and generation of hypobromous acid

To a bottle containing 200 $cm^3$ of Widnes mains water adjusted to pH 8.5, was added 0.2g of 3-bromo-4,4-dimethyl oxazolidin-2-one, the bottle stoppered, and the whole shaken until complete dissolution

of 3-bromo-4,4-dimethyl oxazolidin-2-one had occurred. The solution was then analysed and found to contain 411 mg/l of hypobromous acid. This Example therefore shows that the oxazolidinone dissolves in water to release hypobromous acid which is considered to be the biocidal agent.

Example 3

Biocidal Properties of dilute aqueous solutions of 3-bromo-4,4-dimethyl-oxazolidin-2-one

Three mixed culture water samples taken from industrial cooling towers were exposed for various times to various concentrations of 3-bromo-4,4-dimethyl-oxazolidin-2-one, measured as free hypobromous acid. The samples were then tested for surviving aerobic microbiological species using standard plate count techniques.

| Sample | Contact Time (hours) | Organisms killed (%) for different biocide concentrations | | |
|---|---|---|---|---|
| | | 4 ppm | 10 ppm | 20ppm |
| 1 | 1 | 53.5 | 59.4 | 67.1 |
| | 2 | 69.0 | 73.4 | 79.6 |
| | 4 | 75.0 | 79.3 | 81.6 |

| | | | | |
|---|---|---|---|---|
| | 6 | 75.3 | 78.6 | 82.9 |
| 2 | 0.5 | 98.9 | 97.5 | 99.7 |
| | 1 | 99.5 | 98.3 | 99.8 |
| | 2 | 99.8 | >99.9 | >99.9 |
| | 4 | 99.9 | 99.1 | >99.9 |
| | 6 | 99.9 | 99.2 | >99.9 |
| 3 | 3 | 96.9 | 96.6 | 98.9 |
| | 6 | 96.5 | 97.0 | 99.0 |
| | 24 | 97.9 | 94.9 | >99.0 |

These results demonstrate the biocidal activity of dilute solutions of the oxazolidinone. Its rapid activity and effectiveness is illustrated by, for instance, sample 2

where only 1 in 1,000 microorganisms survived after 4 or 6 hours exposure to 4 ppm of free hypobromous acid.

Example 4

Samples of a mixed - culture water taken from an industrial cooling tower were exposed for various times to 5 ppm of either 3-bromo-4,4-dimethyl-oxazolidin-2-one (I), measured as free hypobromous acid, or of chlorine (II). The samples were then tested for surviving aerobic microbiological species using standard plate count techniques. The results obtained were:-

| Biocide | Contact Time | Organisms killed (%) |
|---|---|---|
| I | 1 hr. | 98.3 |
| | 2 hr. | 99.9 |
| | 4 hr. | 99.9 |
| | 6 hr. | 99.9 |
| II | 1 hr. | 99.5 |
| | 2 hr. | 99.9 |

| | |
|---|---|
| 4 hr. | 99.9 |
| 6 hr. | 99.9 |

This data indicates that the biocidal activity of 3-bromo-4, 4-dimethyl-oxazolidin-2-one is comparable to that of chlorine, which is established as being an effective oxidising biocide.

CLAIMS

1. A method for the treatment of an aqueous system characterized by applying thereto an oxazolidinone of the formula

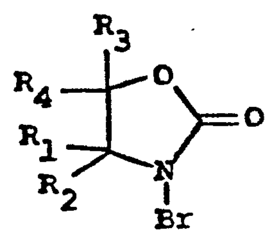

wherein $R_1$, $R_2$, $R_3$ and $R_4$, which may be the same or different, each represents a hydrogen atom or an alkyl radical of one to six carbon atoms.

2. A method according to Claim 1 wherein the said alkyl radical is a methyl radical.

3. A method according to Claim 1 or 2 wherein $R_1$ and $R_2$ both represent a said alkyl radical.

4. A method according to any one of Claims 1 to 3 wherein the oxazolidinone is 3-bromo-4,4-dimethyl-oxazolidin-2-one.

5. A method according to any one of the preceding claims wherein the oxazolidinone is added as a powder alone or with a solid filler.

6. A method according to any one of the preceding claims wherein the oxazolidinone is added in an amount to provide a reserve of from 0.2 to 10 ppm of hypobromous acid above the amount necessary to kill the microorganisms

present.

7. A method according to Claim 6 wherein said amount is 1 to 10 ppm.

8. A method according to any one of the preceding claims wherein the oxazolidinone is added in an amount from 1 to 50 ppm.

9. A method according to any one of the preceding claims for treating an aqueous system used in paper making and the oxazolidinone is added to the paper pulping bath, the recirculating back water or to a holding tank containing the paper pulp.

10. A method according to Claim 9 wherein the oxazolidinone is added together with one or more of starch, titanium dioxide, a de-foamer, a size, a wet strength resin, a polymer dispersant or retention aid, clay, chalk, filler, polyvinyl alcohol, or optical brightening agent.

11. A method according to any one of Claims 1 to 8 wherein the aqueous system is a cooling water system.

12. A method according to Claim 11 wherein the oxazolidinone is in the form of a stick or tablet.

13. A process for preparing an oxazolidinone of the formula as defined in Claim 1 characterised by reacting an alkaline solution of the corresponding unbrominated compound with a solution of bromine in a water immiscible solvent.

14. A process according to Claim 13 in which the solvent is carbon tetrachloride, carbon tetrabromide,

bromoform, benzene, n-hexane, 1,1,2,2,-tetrachloroethane, dichloromethane, chloro bromo-methane, 1,2-dibromoethane, chloroform, carbon disulphide, toluene, 1,1,1-trichloroethane, diethyl ether, dibromomethane or nitrobenzene.

15. A process according to Claim 13 or 14 which comprises adding a solution of bromine in the organic solvent to a cooled solution of the unbrominated oxazolidinone in sodium hydroxide and allowing the desired product to precipitate out.

16. A process according to Claim 15 wherein about two moles of sodium hydroxide are used per one mole of each reactant.

17. A process according to any one of Claims 13 to 16 wherein the solutions used are saturated solutions.

18. A composition suitable for addition to an aqueous system characterised by comprising an oxazolidinone as defined in any one of Claims 1 to 4 together with a carrier.

19. A composition according to Claim 18 wherein the carrier is a solid filler.

20. A composition according to Claim 19 wherein the solid filler is sodium sulphate.

21. A composition according to any one of Claims 18 to 20 which is in the form of a stick or tablet.

22. A composition according to any one of Claims 18 to 21 which also contains one or more of starch, titanium dioxide, a de-foamer, a size, a wet strength resin, a polymer dispersant or retention aid, clay, chalk, polyvinyl alcohol or optical brightening agent.